# EUROPEAN PATENT SPECIFICATION

(11) **EP 3 294 204 B1**
(45) Date of publication and mention of the grant of the patent: **01.05.2019**
(21) Application number: 16724211.4
(22) Date of filing: 09.05.2016
(51) Int. Cl.: A61C 19/06, A61N 5/06, A61C 17/22

(54) **A PERSONAL ORAL-CARE DEVICE**
PERSÖNLICHE MUNDPFLEGEVORRICHTUNG
DISPOSITIF DE SOINS BUCCO-DENTAIRES PERSONNELS

(30) Priority: 15.05.2015 US 201514713794
(43) Date of publication of application: 21.03.2018
(73) Proprietor: The Procter & Gamble Company, Cincinnati, OH 45202 (US)
(72) Inventor: NEWMAN, Matthew, Lloyd, Cincinnati, Ohio 45202 (US); ELLINGSON, Kimberly, Horn, Cincinnati, Ohio 45202 (US); RAJAIAH, Jayanth, Cincinnati, Ohio 45202 (US); SAGEL, Paul, Albert, Cincinnati, Ohio 45202 (US); SCHMID, Franziska, 61476 Kronberg (DE)
(74) Representative: Joos, Uta Susanne
(86) International application number: PCT/US2016/031417
(87) International publication number: WO 2016/186878

(56) References cited:
- CN-Y- 2 902 321
- JP-A- 2005 046 421
- US-A1- 2006 019 214
- US-A1- 2011 189 626
- US-A1- 2012 009 539
- US-A1- 2013 045 457
- US-A1- 2014 315 142
- US-B1- 7 114 257

## Description

### FIELD OF THE INVENTION

The present disclosure is concerned with an oral-care device, and in particular with a personal oral-care electronic device that is structured to prevent its accidental or undesirable activation.

### BACKGROUND OF THE INVENTION

A personal-care electronic device, in particular an electronic device used primarily for oral care, such as, e.g., an electromechanical toothbrush, a light-emitting whitening device, or a heat system for bleaching teeth, must be activated to transfer operation from rest or 'off' state to an active or 'on' state. Typically, a consumer can activate such devices by a single switch. The switch may comprise a mechanical button providing for completion or closing of an electronic circuit by inducement of physical contact of an otherwise broken conducting path. The switch may also comprise elements structured to detect the occurrence of or absence of inductance, capacitance, external conductance, heat, light, sound (or sub-sonic or ultrasonic vibration), or any external state change. A single switch is usually used because of simplicity, convenience, cost, or industrial-design considerations. One switch may cost less than two or more switches, the likelihood of system failure decreases with complexity and switch count, and the complexity of the user interface may increase with increasing switch count.

Accidental or unintended activation of any mechanical equipment may result in several undesirable events, including, e.g., waste of a battery charge, wear and damage of the device, or even hazard to people. Prevention of such an accidental or unintended activation is therefore important. One method to reduce accidental activation of button-controlled heavy machinery is the use of two buttons linked in the activation circuit by a logic gate, such as, e.g., an AND gate. Such an activation circuit requires two buttons to be pressed simultaneously to activate a primary electrical driving circuit of the machinery. Placement of the two switches at such distance as to be unreachable by two fingers or a finger and a thumb of a single hand ensures that an operator's hands are not in close proximity of the machinery during operation. Alternately, the two switches may be placed in series to complete the conducting path of the activation circuit.

In the context of personal battery-operated oral-care devices, the use of multiple switches to occupy user's hands for safety reasons is not typically necessary. On the contrary, it is highly desirable in most cases to have a system that may be easily and instantaneously activated with a single hand, and most typically with a single finger or thumb, e.g. by pressing a single button. But the ease of the single-button activation of a battery-operated personal oral-care device has its negative sides. First, such a system can be prone to accidental activation, e.g., during packing, transportation, and the like events. That would lead to undesirable draining of the battery power. That may also lead to creating a mechanical problem - if the environment in which the accidental activation has occurred prevents the device from operating as designed. In addition, an accidental activation of a device may be hazardous to people, as it would be, e.g., in the hands of a child who is not physically or mentally prepared to properly and safely handle the device.

Hence, the present disclosure is directed to a battery-operated oral-care device that is conveniently activated with a single hand - and that, at the same time, prevents or at least substantially reduces the likelihood of undesired or accidental activation of the device. In particular, the present disclosure is directed to powered oral-care devices in which the electrical power is provided by either a primary, or non-rechargeable, battery cell - or by a rechargeable battery, and in which the prevention of accidental activation during shipping, storage, accident, or other unintended or undesirable use is important.

Light-whitening apparatuses are known in the art. Examples include: US 6,416,319 B1, directed to a device for tooth whitening that has a light source; US 7,572,124 B2, directed to an arrangement for use in whitening a patient's teeth; US 2006/0019214 A1, directed to a compact tooth whitening device; US 2010/0086891 A1, directed to a tooth-whitening apparatus; US 2010/0151407 A1, directed to mouthpieces having activated textured surfaces; and US 2012/0009540 A1, directed to a tooth-whitening device including at least one LED and a removable mouthpiece. US 2011/0189626 A1 discloses a battery operated hand-held teeth whitening system comprising a mouth tray connectable to a separate light housing comprising a light source activable by means of a manual single switch.

US 7,114,257 B1 discloses, in a specific embodiment, an electrical toothbrush comprising a housing and a toothbrush assembly connectable to transmission means comprised in the housing for being driven in oscillation by an electric motor also present in the housing, wherein the housing comprises two switches, located each in a recess and on opposed sides, and wherein the electric motor can be activated with either switch.

### SUMMARY OF THE INVENTION

A personal oral-care device according to claim 1. The first and second switches may be connected either in parallel or in series, depending on a particular electrical configuration of the circuit. The electrical circuit is configured to cause activation of the functional element only after both the first manual switch and the second manual switch have been activated by a user.

The first manual switch and the second manual switch are disposed in a recess within the body sufficiently to prevent an accidental activation of said at least one switch by a substantially planar surface pressing against the body in an area adjacent to said at least one switch. A recess's shape, including a depth, can be configured taking into account ergonomic considerations. For example, the recess may have a radius of curvature or an equivalent thereof of from about 5 mm to about 30 mm. In another embodiment, the recess may have a depth of from about 5 mm to about 30 mm.

The body of the device has a first side and a second side opposite to the first side, wherein the first manual switch is disposed on the first side and the second manual switch is disposed on the second side. The first manual switch is disposed in a first recess formed on the first side of the body, and the second manual switch is disposed in a second recess formed on the second side of the body. The first recesses advantageously has at least one dimension (such as length, width, or depth) that is greater than a corresponding dimension of the second recess. Such a configuration is particularly beneficial in the device intended to be activated by a thumb and one other finger of a user's hand, wherein the first recess can conveniently accommodate the thumb.

In one embodiment of the device, one or both of the first and second manual switches may be structured as a push button configured to be pressed by a user's finger or thumb to activate the device. A finger-contact surface of the first manual switch and/or the second manual switch can have any suitable, preferably ergonomically friendly, shape. As used herein, a finger-contact surface includes a thumb-contact surface. In one embodiment at least one of the first and second manual switches has a concave finger-contact surface. In another embodiment, at least one of the first and second manual switches has a concave finger-contact surface.

In one embodiment, the oral-care device is a tooth-whitening apparatus comprising at least one light source structured and configured to provide illumination of user's front teeth upon activation of the device. Such a tooth-whitening apparatus may include an optical element comprising an interface between the light source and the user's teeth. The optical interface can be advantageously structured and configured to provide a substantially uniform illumination of the user's front teeth upon activation of the device. Examples of the optical interface include transparent or translucent thermoplastics, transparent or translucent thermoplastic elastomers, transparent or translucent silicone rubbers, transparent or translucent glasses, and any combination thereof.

The light source or sources can be any suitable element, e.g., light-emitting diodes, e.g., in the infrared, visible, or ultraviolet range of frequency. Other suitable light sources such as, e.g., lasers in the infrared, visible, or ultraviolet range of frequency can be used as well, in various combinations, depending on the application.

The tooth-whitening apparatus may also include a dental tray structured and configured to receive a tooth-whitening composition therein. The dental tray can have a generally arched shape and configured to fit user's front teeth.

In one embodiment, the tooth-whitening apparatus comprises a timer structured to deactivate the device automatically after a predetermined period of time from activation of the device has expired.

### BRIEF DESCRIPTION OF THE DRAWINGS

While the specification concludes with claims which particularly point out and distinctly claim the subject matter that is regarded as the invention, the various embodiments will be better understood from the following description taken in conjunction with the accompanying drawings, in which:
Fig. 1 is a schematic perspective view of an embodiment of an oral-care device.
Fig. 2 is a schematic plan view of the device shown in Fig. 1.
Fig. 3 is a schematic plan view of another embodiment of the oral-care device.
Fig. 4 is a schematic plan view of yet another embodiment of the oral-care device.
Fig. 5 is a schematic side view of the device shown in Fig. 4.
Fig. 6 is a schematic plan view of another embodiment of the oral-care device.
Fig. 7 is a schematic diagram of an embodiment of an electrical circuit that can be used in the device, wherein switches are in parallel.
Fig. 8 is a schematic diagram of an embodiment of an electrical circuit that can be used in the device, wherein switches are in series.

### DETAILED DESCRIPTION OF THE INVENTION

A personal oral-care device 10 of the disclosure comprises a functional element 20 and a battery 30 supplying energy to the functional element 20 to cause the functional element 20 to perform at least one oral-care function. The personal oral-care device 10 may comprise any electrical or electromechanical device, such as, e.g., a light-emitting tooth-whitening device, a heat source for bleaching teeth, and the like. The at least one oral-care function can be selected from any known operations of these exemplary devices, such as, e.g., tooth brushing, tooth whitening, tooth bleaching, and the like. In one exemplary embodiment, described herein in detail, the device 10 comprises a tooth-whitening apparatus. The functional element 20 of the tooth-whitening apparatus includes at least one light source structured and configured to provide illumination of user's front teeth upon activation of the device 10.

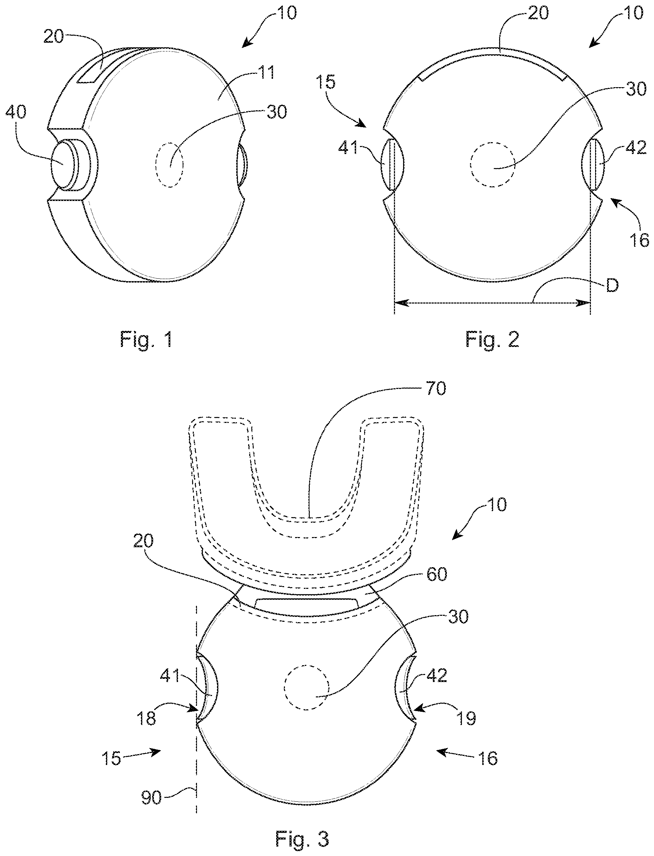

As is shown in Figs. 1-4, an embodiment of the device 10 comprises a body 11 that houses the battery 30 therein. The device 10 includes at least two manual switches 40 disposed on the body: a first manual switch 41 and a second manual switch 42. The switches 41, 42 are disposed at a distance D from one another. This distance D, in combination with a shape of the body 11, is calculated to prevent simultaneous activation of the first and second manual switches 41, 42 with a single finger or thumb of a user or a single substantially planar surface 90 (Fig. 3). This is designed to avoid accidental or unintended activation of the device 10. In one embodiment of the device, the distance D between the first manual switch 41 and the second manual switch 42 is from 10 mm to 100 mm. In another embodiment, the distance D between the first manual switch 41 and the second manual switch 42 is from 20 mm to 80 mm. In yet another embodiment, the distance D between the first manual switch 41 and the second manual switch 42 is from 30 mm to 70 mm.

The device 10 includes an electrical circuit 50, supplying power from the battery 30 to the functional element 20. The first and second switches 41 and 42 are naturally part of the electrical circuit 50. Any suitable design of the switches 41, 42 may be used in the device 10. Either switch 41, 42 may comprise, e.g., a mechanical button providing for completion or closing of the electronic circuit 50 by inducement of physical contact of an otherwise-broken conducting path. Either switch 41, 42 may also comprise elements structured and configured to detect the occurrence or absence of inductance, capacitance, external conductance, heat, light, sound, sub-sonic or ultrasonic vibration, or any other change of an external state.

Any one of the switches 41, 42 may be structured as a latching switch or a momentary switch. A latching switch is a switch that requires a single, temporary activation by a user to change state permanently, but reversibly, from the ON (or closed) state to the OFF (or open) state. One example of a latching switch is the common household light switch used to open and close main power (110VAC in the US and 220VAC in Europe) to a circuit comprising a conducing path and one or more light bulbs. A latching switch is typically a simple mechanical system that usually requires no additional electronic elements for control or timing.

A momentary switch is a switch that closes a conducting path only temporarily during some user-activated state. It is often used for convenience, cost, or industrial-design considerations where a latching switch may not be desired. A momentary switch may, e.g., comprise a button that leaves a conducting path in an electronic circuit open during the "up" position and closes said conducting path when depressed, or during the "down" position. One example of a momentary switch is an elevator button, which, when depressed, completes the conducting path comprising a small ring of light around the button of the desired floor. The lighted ring remains lit even when the button is released by the user. Such a switch may be combined with transistors, microcontrollers, and other electronic elements to provide for continued operation of an electronic device even when said switch is released to the "up" position. In this case, the momentary switch controls an activating circuit which serves only to initiate - but not continue driving - the primary electrical driving circuit. Advantages of a momentary switch are cost and simplicity of the individual switch component design; however, in a system whose primary electrical driving circuit must be active for more than a moment, or must be active while the switch is open, the electronics necessary to connect the activating circuit to the primary electrical driving circuit typically outweigh the cost or simplicity advantages of a momentary switch.

A primary disadvantage of both momentary and latching switches that are designed to be activated by a user is that they can be turned on accidentally - which often leads to an unintended activation of the driving electrical circuit and consequently of the functional element controlled by the driving electrical circuit. For example, the button on an electromechanical toothbrush may be accidentally depressed during placement of the toothbrush into a loose storage in a bathroom drawer. As further examples, the activation button on a light-emitting device may be depressed during rough handling during shipment, or as a result of dropping or placing of the device onto, e.g., a bathroom-counter surface, such that an impact resulting from a contact between the activation button and the surface causes activation of the device. Occurrences like these are highly undesirable, for they may lead to unintended and wasteful battery consumption.

Furthermore, accidental or unintended activation of some electromechanical devices may cause discomfort, particularly for children. For example, the intense light emitted by the tooth-whitening device may, in some instances, be uncomfortable to the human eyes, and particularly the eyes of a child - who may not instantaneously appreciate the discomfort of a prolonged exposure to such a light. In cases where ultraviolet light, in particular, is emitted from a device, safety is of a paramount concern, as the human eye cannot detect the presence of UV light, yet the eye can be damaged by such light. Then, features to mitigate accidental discharge of UV light must be required to ensure safe use. In such instances, a separate system including light in the visible range should be included in parallel with the UV-emitting system, to indicate UV discharge when the device is in the "ON" state.

Accidental activation of switches is not confined to those switches activated by mechanical buttons. Switches may be activated by the occurrence of or absence of inductance, capacitance, external conductance, heat, light, sound (or sub-sonic or ultrasonic vibration), or any external-state change. The touch screen on a modern cellphone is typically an array of capacitive elements activating switches, said capacitive elements sensitive to both intended activation by touch of a finger or thumb, or accidental activation by touch of an earlobe during hand-held conversational use.

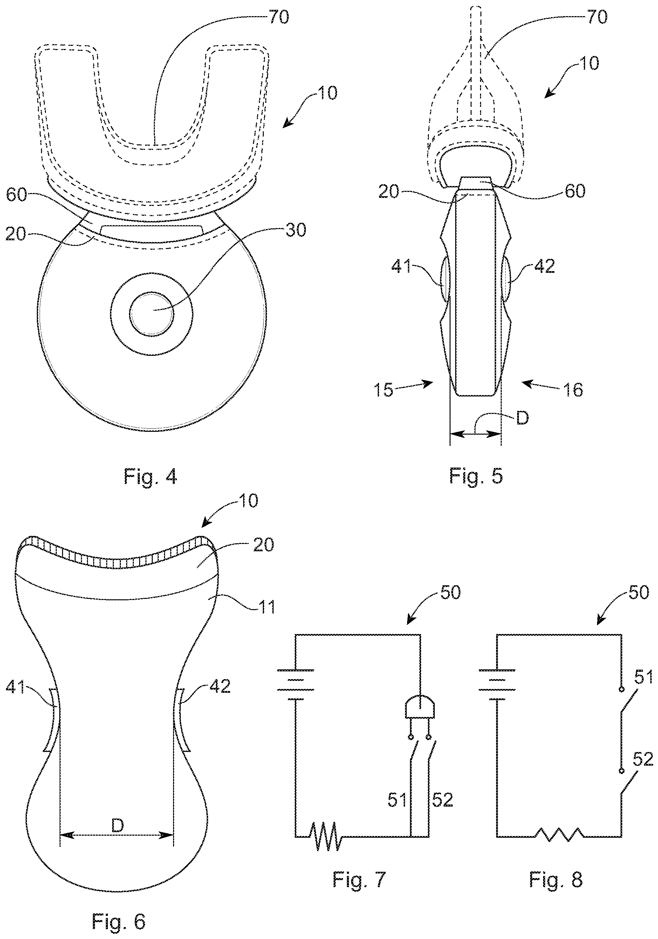

The electrical circuit 50 can be structured and configured to conduct the current path that drives the functional element 20 of the device 10. The electrical circuit 50 can comprise, e.g., an electromechanical drive system of a toothbrush (not shown), or to cause a light emission from light-emitting diodes (LEDs) of a tooth-whitening device (Figs. 1-4), or to cause infrared heat generation in a heat system of a tooth-bleaching device. Any suitable configuration of the electrical circuit 50 can be used in the device 10. For example, Fig. 7 shows an embodiment wherein the first and second switches 41, 42 are included in parallel, while Fig. 8 shows an embodiment wherein the switches 41, 42 are included in series. Regardless of the particular electrical configuration, the device 10 can be activated only when both the first switch 41 and the second switch 42 are in the state of simultaneous activation prompted by a user of the device 10. By "the state simultaneous activation" it is meant that both the first and second switches 41, 42 are caused to be in an ON state at any fraction of time, although the first switch 41 can be initiated or activated (i.e., touched or pressed) in a first instant and the second switch 42 can be initiated or activated (i.e., touched or pressed) in a second instant different in time from the first instant.

At least one of the two manual switches 41, 42 can be advantageously recessed within the body 11 sufficiently to prevent an accidental activation of said at least one switch by a substantially planar surface 90 pressing against the body in an area adjacent to said at least one switch. Such a configuration may be particularly beneficial in situations when the device is expected to be placed on a surface, such as, e.g., a surface of a bathroom counter, and the like, or accidentally dropped on the floor. If the switch 40 is recessed within the body 11 sufficiently to avoid contact with the flat surface when the flat surface contacts the body surrounding the switch 40, accidental activation of the button is virtually excluded.

In the embodiment of Fig. 3, the first manual switch 41 is disposed in a first recess 18 formed on the first side 15 of the body 11, and the second manual switch 42 is disposed in a second recess 19 formed on the second side 16 of the body 11. The first recesses 18 may advantageously have a suitable shape and at least one dimension (such as length, width, or depth) that is greater than a corresponding dimension of the second recess 19. Such a configuration may be particularly beneficial in the device 10 intended to be activated by a thumb and one other finger of a user's hand, wherein the first recess can conveniently accommodate the thumb.

One skilled in the art would be able to envision any number of specific embodiments of the device 10 having two manual switches 40 as is disclosed herein. The device 10 may have, e.g., a first side 15 and a second side 16 opposite the first side 15, so that the first manual switch 41 is disposed on the first side 15 and the second manual switch 42 is disposed on the second side 16, Figs. 2, 5. In both exemplary embodiments of Figs. 2, 5, each of the first manual switch 41 and the second manual switch 42 is shown as a push button structured and configured to be pressed or otherwise moved by a user's finger or thumb to activate the device 10. But one skilled in the art will readily recognize that any suitable design of the manual switch can be used in the device 10, e.g., touch-screen buttons and switches comprising capacitive elements.

In further embodiments of the device 10, the manual switches 41, 42 may have any finger-contact surface, preferably ergonomically friendly and /or aesthetically pleasing to a user of the device. For example, at least one of the manual switches 41, 42 may have a finger-contact surface that is concave (Figs. 3, 6) or convex (Figs. 2, 5).

The tooth-whitening apparatus may further have an optical interface 60 located between the light source and the user's teeth and structured to provide a substantially uniform illumination of the user's front teeth upon activation of the device. The optical interface can comprise, e.g., transparent or translucent thermoplastics, transparent or translucent thermoplastic elastomers, transparent or translucent silicone rubbers, transparent or translucent glasses, and any combination thereof, as is known in the art. The optical interface may be an integral part of the device 10 or may be incorporated in a replaceable mouthpiece 70, Fig. 3, 4. The mouthpiece 70 can be structured and configured to assist a user in proper positioning of the device relative to the user's teeth and in giving the optical interface a correct orientation relative the user's teeth for a maximal whitening effect. The mouthpiece 70 may also comprise a dental tray 70 and may conveniently have a working surface having a generally arched shape to approximately match the overall shape of the front teeth. The dental tray 70 may also be structured to contain a tooth-whitening composition, as is known in the art. The tooth-whitening composition may be a strip, a gel, or paste.

In a further embodiment, the device 10 can be equipped with a timer structured to deactivate the device 10 automatically after a predetermined period of time from the activation of the device.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "10 µm" is intended to mean "about 10 µm."

Every document cited herein, including any cross-referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A personal oral-care device (10) comprising a functional element (20) and a battery (30) supplying energy thereto to cause the functional element (20) to perform at least one oral-care function, the device (10) comprising:
a body (11) containing the battery (30);
a first manual switch (41) disposed on the body (11);
a second manual switch (42) disposed on the body (11) and at a distance (D) from the first manual switch (41), wherein the distance (D) between the first and second manual switches (41, 42) is from 10 mm to 100 mm and wherein the first manual switch (41) is disposed in a first recess (18) formed on a first side (15) of the body (11), and the second manual switch (42) is disposed in a second recess (19) formed on a second side (16) of the body (11), the first side (15) substantially opposite to the second side (16), and wherein the first recess (18) has at least one dimension that is greater than a corresponding dimension of the second recess (19);
and an electrical circuit (50) supplying power from the battery (30) to the functional element (20) and including the first and second manual switches (41, 42),
wherein the electrical circuit (50) is configured to cause activation of the functional element (20) only after both the first manual switch (41) and the second manual switch (42) have been caused to be in a state of simultaneous activation.

2. The device (10) according to the preceding claim, wherein at least one of the first manual switch (41) and the second manual switch (42) comprises a push button, wherein at least one of the first manual switch (41) and the second switch (42) comprises a finger-contact surface that is concave or a finger-contact that is convex.

3. The device (10) according to anyone of the preceding claims, wherein the first manual switch (41) and the second manual switch (42) are connected in series or are connected in parallel.

4. The device (10) according to anyone of the preceding claims, wherein the recesses (18, 19) have a radius of curvature or an equivalent thereof and/or a depth of from about 5 mm to about 30 mm.

5. The device (10) according to anyone of the preceding claims, wherein the at least one dimension of the recesses (18, 19) is selected from the group consisting of depth, length, and width of the recesses (18, 19).

6. The device (10) according to anyone of the preceding claims, wherein the distance (D) between the first manual switch (41) and the second manual switch (42) is from 20 mm to 80 mm, preferably from 30 mm to 70 mm.

7. The device (10) according to anyone of the preceding claims, wherein the device (10) is a tooth-whitening apparatus comprising at least one light source being the functional element (20) structured and configured to provide illumination of user's front teeth upon activation of the device (10).

8. The device (10) according to claim 7, wherein the tooth-whitening apparatus further comprises an optical interface (60) between the light source and the user's teeth, the optical interface (60) being structured and configured to provide a substantially uniform illumination of the user's front teeth upon activation of the device (10).

9. The device (10) according to anyone of claims 7 or 8, wherein the light source is selected from the group consisting of light-emitting diodes, in the infrared, visible, or ultraviolet range of frequency; lasers in the infrared, visible, or ultraviolet range of frequency, and any combination thereof.

10. The device (10) according to anyone of claims 8 or 9, wherein the optical interface (60) is selected from the group consisting of transparent or translucent thermoplastics, transparent or translucent thermoplastic elastomers, transparent or translucent silicone rubbers, transparent or translucent glasses, and any combination thereof.

11. The device (10) according to anyone of claims 7 to 10, wherein the tooth-whitening apparatus further comprises at least one dental tray (70) having a generally arched shape, the dental tray (70) being structured to contain a tooth-whitening composition and configured to fit user's front teeth.

12. The device (10) according to anyone of claims 7 to 11, wherein the tooth-whitening apparatus comprises a timer structured to deactivate the device (10) automatically after a predetermined period of time from activation of the device (10).

## Patentansprüche

1. Mundpflegevorrichtung (10) für den persönlichen Gebrauch, die ein Funktionselement (20) und eine ihr Energie zuführende Batterie (30) umfasst, um zu bewirken, dass das Funktionselement (20) mindestens eine Mundpflegefunktion ausführt, wobei die Vorrichtung (10) Folgendes umfasst:
einen Körper (11), der die Batterie (30) enthält;
einen ersten manuellen Schalter (41), der an dem Körper (11) angeordnet ist;
einen zweiten manuellen Schalter (42), der an dem Körper (11) und in einem Abstand (D) von dem ersten manuellen Schalter (41) angeordnet ist, wobei der Abstand (D) zwischen dem ersten und dem zweiten manuellen Schalter (41, 42) von 10 mm bis 100 mm beträgt, und wobei der erste manuelle Schalter (41) in einer ersten Aussparung (18) auf einer ersten Seite (15) des Körpers (11) angeordnet ist, und der zweite manuelle Schalter (42) in einer zweiten Aussparung (19) auf einer zweiten Seite (16) des Körpers (11) angeordnet ist, wobei die erste Seite (15) der zweiten Seite (15) im Wesentlichen gegenüberliegt, und wobei die erste Aussparung (18) mindestens eine Abmessung aufweist, die größer ist als eine entsprechende Abmessung der zweiten Aussparung (19);
und eine elektrische Schaltung (50), die Leistung von der Batterie (30) an das Funktionselement (20) liefert und die ersten und zweiten manuellen Schalter (41, 42) einschließt,
wobei die elektrische Schaltung (50) dafür konfiguriert ist, eine Aktivierung des Funktionselements (20) nur dann zu bewirken, nachdem sowohl der erste manuelle Schalter (41) als auch der zweite manuelle Schalter (42) in einem Zustand gleichzeitiger Aktivierung gebracht wurden.

2. Vorrichtung (10) nach dem vorstehenden Anspruch, wobei mindestens einer von dem ersten manuellen Schalter (41) und dem zweiten manuellen Schalter (42) einen Druckknopf umfasst, wobei mindestens einer von dem ersten manuellen Schalter (41) und dem zweiten manuellen Schalter (42) eine Fingerkontaktoberfläche umfasst, die konkav ist, oder einen Fingerkontakt, der konvex ist.

3. Vorrichtung (10) nach einem der vorstehenden Ansprüche, wobei der erste manuelle Schalter (41) und der zweite manuelle Schalter (42) in Reihe geschaltet oder parallel geschaltet sind.

4. Vorrichtung (10) nach einem der vorstehenden Ansprüche, wobei die Aussparungen (18, 19) einen Krümmungsradius oder ein Äquivalent davon und/oder eine Tiefe von etwa 5 mm bis etwa 30 mm aufweisen.

5. Vorrichtung (10) nach einem der vorstehenden Ansprüche, wobei die mindestens eine Abmessung der Aussparungen (18, 19) ausgewählt ist aus der Gruppe bestehend aus Tiefe, Länge und Breite der Aussparungen (18, 19).

6. Vorrichtung (10) nach einem der vorstehenden Ansprüche, wobei der Abstand (D) zwischen dem ersten manuellen Schalter (41) und dem zweiten manuellen Schalter (42) von 20 mm bis 80 mm, vorzugsweise von 30 mm bis 70 mm beträgt.

7. Vorrichtung (10) nach einem der vorstehenden Ansprüche, wobei die Vorrichtung (10) eine Zahnaufhellungsvorrichtung ist, die mindestens eine Lichtquelle umfasst, die das Funktionselement (20) ist, das dafür strukturiert und konfiguriert ist, bei Aktivierung der Vorrichtung (10) eine Beleuchtung der Vorderzähne des Benutzers bereitzustellen.

8. Vorrichtung (10) nach Anspruch 7, wobei die Zahnaufhellungsvorrichtung ferner eine optische Schnittstelle (60) zwischen der Lichtquelle und den Zähnen des Benutzers umfasst, wobei die optische Schnittstelle (60) dafür strukturiert und konfiguriert ist, bei Aktivierung der Vorrichtung (10) eine im Wesentlichen gleichförmige Beleuchtung der Vorderzähne des Benutzers bereitzustellen.

9. Vorrichtung (10) nach einem der Ansprüche 7 oder 8, wobei die Lichtquelle ausgewählt ist aus der Gruppe bestehend aus Leuchtdioden im infraroten, sichtbaren oder ultravioletten Frequenzbereich; Lasern im infraroten, sichtbaren oder ultravioletten Bereich der Frequenz, und jede Kombination davon.

10. Vorrichtung (10) nach einem der Ansprüche 8 oder 9, wobei die optische Schnittstelle (60) ausgewählt ist aus der Gruppe bestehend aus transparenten oder transluzenten Thermoplasten, transparenten oder transluzenten Thermoplastelastomeren, transparenten oder transluzenten Silikonkautschuken, transparenten oder transluzenten Gläsern und jeder Kombination davon.

11. Vorrichtung (10) nach einem der Ansprüche 7 bis 10, wobei die Zahnaufhellungsvorrichtung ferner mindestens eine Zahnschiene (70) mit einer im Allgemeinen bogenförmigen Form umfasst, wobei die Zahnschiene (70) dafür strukturiert ist, dass sie eine Zahnaufhellungszusammensetzung enthält, und die dafür konfiguriert ist, dass sie den Vorderzähnen des Benutzers entspricht.

12. Vorrichtung (10) nach einem der Ansprüche 7 bis 11, wobei die Zahnaufhellungsvorrichtung einen Zeitgeber umfasst, der dafür strukturiert ist, dass er die Vorrichtung (10) nach einer vorbestimmten Zeitdauer automatisch von der Aktivierung der Vorrichtung (10) deaktiviert.

## Revendications

1. Dispositif personnel de soins bucco-dentaires (10) comprenant un élément fonctionnel (20) et une batterie (30) l'alimentant en énergie pour amener l'élément fonctionnel (20) à effectuer au moins une fonction de soins bucco-dentaires, le dispositif (10) comprenant :
un corps (11) contenant la batterie (30) ;
un premier commutateur manuel (41) disposé sur le corps (11) ;
un deuxième commutateur manuel (42) disposé sur le corps (11) et à une distance (D) du premier commutateur manuel (41), dans lequel la distance (D) entre les premier et deuxième commutateurs manuels (41,42) va de 10 mm à 100 mm et dans lequel le premier commutateur manuel (41) est disposé dans un premier renfoncement (18) formé sur un premier côté (15) du corps (11), et le deuxième commutateur manuel (42) est disposé dans un deuxième renfoncement (19) formé sur un deuxième côté (16) du corps (11), le premier côté (15) essentiellement opposé au deuxième côté (16), et dans lequel le premier renfoncement (18) a au moins une dimension qui est plus grande qu'une dimension correspondante du deuxième renfoncement (19) ;
et un circuit électrique (50) alimentant en énergie à partir de la batterie (30) l'élément fonctionnel (20) et incluant les premier et deuxième commutateurs manuels (41, 42),
dans lequel le circuit électrique (50) est configuré pour amener une activation de l'élément fonctionnel (20) uniquement après que l'un et l'autre du premier commutateur manuel (41) et du deuxième commutateur manuel (42) ont été amenés à se trouver dans un état d'activation simultanée.

2. Dispositif (10) selon la revendication précédente, dans lequel au moins l'un du premier commutateur manuel (41) et du deuxième commutateur manuel (42) comprend un bouton poussoir, dans lequel au moins l'un du premier commutateur manuel (41) et du deuxième commutateur (42) comprend une surface de contact avec le doigt qui est concave ou un contact avec le doigt qui est convexe.

3. Dispositif (10) selon l'une quelconque des revendications précédentes, dans lequel le premier commutateur manuel (41) et le deuxième commutateur manuel (42) sont connectés en série ou sont connectés en parallèle.

4. Dispositif (10) selon l'une quelconque des revendications précédentes, dans lequel les renfoncements (18, 19) ont un rayon de courbure ou équivalent de celui-ci et/ou une profondeur allant d'environ 5 mm à environ 30 mm.

5. Dispositif (10) selon l'une quelconque des revendications précédentes, dans lequel l'au moins une dimension des renfoncements (18, 19) est choisie dans le groupe constitué de profondeur, longueur et largeur des renfoncements (18, 19).

6. Dispositif (10) selon l'une quelconque des revendications précédentes, dans lequel la distance (D) entre le premier commutateur manuel (41) et le deuxième commutateur manuel (42) va de 20 mm à 80 mm, de préférence de 30 mm à 70 mm.

7. Dispositif (10) selon l'une quelconque des revendications précédentes, dans lequel le dispositif (10) est un appareil de blanchiment des dents comprenant au moins une source de lumière étant l'élément fonctionnel (20) structuré et configuré pour fournir un éclairage des dents avant d'un utilisateur lors de l'activation du dispositif (10).

8. Dispositif (10) selon la revendication 7, dans lequel l'appareil de blanchiment des dents comprend en outre une interface optique (60) entre la source de lumière et les dents de l'utilisateur, l'interface optique (60) étant structurée et configurée pour fournir un éclairage essentiellement uniforme des dents avant de l'utilisateur lors de l'activation du dispositif (10).

9. Dispositif (10) selon l'une quelconque des revendications 7 ou 8, dans lequel la source de lumière est choisie dans le groupe constitué de diodes électroluminescentes, dans la plage de fréquence infrarouge, visible ou ultraviolette ; de lasers dans la plage de fréquence infrarouge, visible ou ultraviolette, et de n'importe quelle combinaison de ceux-ci.

10. Dispositif (10) selon l'une quelconque des revendications 8 ou 9, dans lequel l'interface optique (60) est choisie dans le groupe constitué de thermoplastiques transparents ou translucides, élastomères thermoplastiques transparents ou translucides, caoutchoucs de silicone transparents ou translucides, verres transparents ou translucides, et n'importe quelle combinaison de ceux-ci.

11. Dispositif (10) selon l'une quelconque des revendications 7 à 10, dans lequel l'appareil de blanchiment des dents comprend en outre au moins une gouttière dentaire (70) ayant une forme généralement arquée, la gouttière dentaire (70) étant structurée pour contenir une composition de blanchiment des dents et configurée pour s'ajuster aux dents avant de l'utilisateur.

12. Dispositif (10) selon l'une quelconque des revendications 7 à 11, dans lequel l'appareil de blanchiment des dents comprend une minuterie structurée pour désactiver le dispositif (10) automatiquement après une période de temps prédéterminée à partir de l'activation du dispositif (10).
